(19) **Europäisches Patentamt**

**European Patent Office**

**Office européen des brevets**

(11) Veröffentlichungsnummer: **0 297 239 B1**

(12) # EUROPÄISCHE PATENTSCHRIFT

(45) Veröffentlichungstag der Patentschrift: **31.03.93**

(51) Int. Cl.⁵: **C07C 33/04**, C07C 29/76

(21) Anmeldenummer: **88106621.1**

(22) Anmeldetag: **26.04.88**

(54) **Verfahren zur Herstellung von Hexadiin(2,4)-diol-1,6.**

(30) Priorität: **30.06.87 DE 3721474**

(43) Veröffentlichungstag der Anmeldung:
**04.01.89 Patentblatt 89/01**

(45) Bekanntmachung des Hinweises auf die
Patenterteilung:
**31.03.93 Patentblatt 93/13**

(84) Benannte Vertragsstaaten:
**BE DE FR GB IT NL**

(56) Entgegenhaltungen:
**EP-A- 0 168 893**
**US-A- 4 620 911**

(73) Patentinhaber: **HÜLS AKTIENGESELLSCHAFT**
**- RSP Patente / PB 15 - Postfach 13 20**
**W-4370 Marl 1(DE)**

(72) Erfinder: **Sridhar, Srinivasan, Dr.**
**Lehmbecker Pfad 52**
**W-4370 Marl(DE)**
Erfinder: **Westernacher, Helmut, Dr.**
**Burgstrasse 21**
**W-4358 Haltern(DE)**

Rank Xerox (UK) Business Services
(3.10/3.5x/3.0.1)

**Beschreibung**

Die Herstellung von Hexadiin(2,4)-diol-1,6 kann durch Ethinylierung von Diacetylen mit Formaldehyd erfolgen. Wegen des hohen Gefahrenpotentials des Diacetylens (Neigung zu explosionsartiger Zersetzung), wird der Umgang mit dieser Verbindung nur in hoher Verdünnung empfohlen. Ein wirtschaftlich sinnvolles Produktionsverfahren ist in dieser Weise kaum zu verwirklichen.

Hexadiin(2,4)-diol-1,6 kann aber auch auf weit ungefährlicherem Wege durch oxidative Kupplung nach Glaser aus Propargylalkohol hergestellt werden. L. Brandsma beschreibt in "Preparative Acetylenic Chemistry" Elsevier Publishing Company, Amsterdam, 1971, Seite 166, ein entsprechendes Verfahren. Der Nachteil dieses dort beschriebenen Verfahrens ist der, daß das Hexadiindiol, da relativ schlecht wasserlöslich, aus dem Reaktionsgemisch ausfällt. Die Aufarbeitung eines solchen Kristallbreies ist bekanntlich sehr aufwendig.

Zudem enthält der Kristallbrei noch Salze vom Katalysator für die oxidative Kupplung von Propargylalkohol. Da Hexadiin(2,4)-diol-1,6 in Wasser, wenn auch wenig, löslich ist, kann eine Wasserwäsche nur mit Produktverlust angewandt werden. Eine destillative Abtrennung ist ebenfalls ungeeignet, da Hexadiin(2,4)-diol-1,6 thermisch labil ist und im Vakuum höher siedet als Wasser.

Der geläufige Weg über Ionenaustauscher eignet sich in einem solchen Fall, wenn der Salzgehalt nicht allzu hoch ist. Jedoch ist bei einer Regenerierung des Austauschers mit salzhaltigen Eluaten zu rechnen, die nicht nur einen Produktverlust, sondern auch eine Umweltbelastung herbeiführen. Eine Entsalzung durch Umkehrosmose käme hier auch nicht in Frage: Die Beständigkeit der Membrane sowie der Permeatfluß in organischen Medien werfen Probleme auf, und das salzhaltige Retentat bedarf weiterer Aufarbeitung.

Hieraus ergab sich die Aufgabe, ein Verfahren zu finden, das es ermöglicht, Hexadiin(2,4)-diol-1,6 in wirtschaftlicher Weise herzustellen, wobei in der Aufarbeitung die Salze entfernt werden.

Diese Aufgabe wird erfindungsgemäß entsprechend den Angaben der Patentansprüche gelöst.

Überraschenderweise kann man das Ausfällen des entstandenen Hexadiindiol-1,6 vermeiden, wenn man der Reaktionsmischung Butanol zusetzt. Es bildet sich ein heterogenes Gemisch von zwei Phasen, wobei sich das Hexadiindiol-1,6 praktisch vollständig im Butanol löst. Nach Beendigung der Reaktion und Abstellen des Rührers trennt sich die butanoische Lösung des Hexadiindiols schnell und sauber von der wäßrigen Katalysatorlösung durch Phasentrennung.

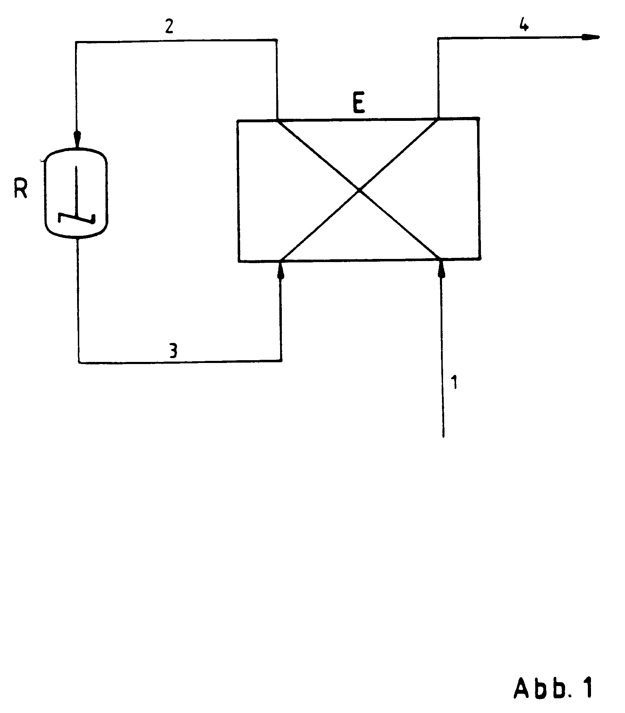

Als Butanol sind n-, iso- und tert.-Butanol geeignet. Das Verhältnis Katalysatorphase zu Butanolphase liegt bei 5 : 1 bis 1 : 5, vorzugsweise bei 3 : 1 bis 1 : 2, insbesondere bei ca. 2 : 1. Bevorzugt trägt man in die wäßrige Katalysatorlösung unter Rühren eine Lösung von 5 bis 50 %, insbesondere von 10 bis 30 % Propargylalkohol in Butanol und gegebenenfalls Wasser ein (Strom 2 in Abb. 1).

Als Katalysator setzt man im allgemeinen Kupfersalz-Komplexe in wäßriger Lösung ein, beispielsweise einen $Cu^ICl/NH_4Cl$-Komplex ein.

Die Umsetzung erfolgt im allgemeinen in einem mit Rührer, Druckhalteventil und Thermometer ausgerüsteten Druckgefäß (Reaktor R in Abb. 1), in das man die wäßrige Katalysatorlösung gibt, die man beispielsweise herstellt aus etwa 2 bis 8 % Kupfer(I)chlorid, etwa 10 bis 26 % Ammoniumchlorid und etwa 66 bis 88 % Wasser. Katalysatorlösungen mit geringer Salzkonzentration sind ungünstig, da sie die Reaktionszeit unwirtschaftlich lange verlängern bzw. die oxidative Kupplung überhaupt nicht mehr katalysieren.

Das Reaktionsgemisch bringt man auf die Reaktionstemperatur von 5 bis 80 °C, bevorzugt 10 bis 50 °C, und leitet die Glaser-Kupplung durch Aufdrücken von Sauerstoff ein. Die Reaktionswärme wird durch Kühlung entfernt. Den Druck stellt man auf 0,1 bis 5 bar Überdruck, bevorzugt 0,5 bis 3 bar Überdruck, ein. Anstelle von reinem Sauerstoff kann auch Luft verwendet werden. In diesem Falle muß jedoch zur Aufrechterhaltung der Sauerstoffkonzentration ein kontinuierlicher Abgasstrom aus dem Reaktor sichergestellt werden.

Die Reaktion ist beendet, wenn der Propargylalkoholgehalt auf < 0,5 % abgesunken ist.

Das Verfahren kann man diskontinuierlich oder kontinuierlich durchführen.

Der Reaktor wird dann entspannt, der Rührer ausgeschaltet und die sich oben abscheidende Butanolphase ausgetragen. Die Katalysatorlösung bleibt im Reaktor und kann zu weiteren Umsetzungen verwendet werden.

Die Butanolphase enthält noch Restmengen an Salzen von ca. 0,1 bis 2 Gewichtsprozent. Die Entfernung der Restmengen an Salzen erfolgt erfindungsgemäß mit Hilfe einer Elektrodialyse, wobei man Butanol, ein systemeigenes, produktfreies, organisches Lösemittel mit wenig Wasser und Propargylalkohol, als Aufnehmerlösung einsetzt, wodurch die Salze aus dem Reaktionsgemisch abgetrennt und mit dem Aufnehmer zum katalytischen Einsatz der Reaktion zurückgeführt werden können. Damit erfolgt sowohl die Entsalzung als auch die Rückgewinnung der Salze ohne Umweltbelastung.

Das Reaktionsgemisch führt man aus dem Reaktor R als Strom 3 (Abb. 1) in die Elektrodialyse E, in der man es elektrodialytisch, beispielsweise in einem Membranstapel E, von den Salzen befreit. Als Membrane kann man handelsübliche Membrane einsetzen, vorzugsweise mit einer Kammerdicke von 0,3 bis 3 mm und einer Membrandicke von 0,1 bis 0,3 mm.

Die Membrane bestehen im allgemeinen aus Styrol-Divinylbenzol mit angelagerten funktionellen Gruppen auf Gewebe aus Polyvinylchlorid (bei Kationenaustauschmembran) bzw. Polypropylen (Anionenaustauschmembran). Die Kationen- bzw. Anionenaustauschaktivität wird z. B. durch angelagerte Sulfonat- bzw. quaternäre Amino-Gruppen bewerkstelligt.

Je Kammerpaar legt man im allgemeinen eine Spannung von 2 bis 10 V. Die spezifische Leitfähigkeit des Ausgangsgemisches beträgt im allgemeinen 400 bis 1000 [$\mu$S/cm], die Stromdichte 1 bis 50 [mA/cm$^2$]. Während der Elektrodialyse nimmt die spezifische Leitfähigkeit und damit auch die Stromstärke ab. Die Elektrodialyse führt man im allgemeinen bei niedrigen Temperaturen, vorzugsweise bei 10 bis 40 °C, insbesondere bei 20 bis 30 °C, durch.

Der Elektrodenstapel besteht aus alternierenden Anion- und Kationenaustauschmembranen, die zwischen zwei Elektroden angebracht werden. Die Elektroden können mit Säure, Lauge bzw. einer Salzlösung umspült werden. Zwei Membrane bilden eine Kammer. Zwei benachbarte Kammern, die von Aufnehmer- bzw. Abgeberlösungen beschickt werden, bilden als Kammerpaar eine funktionelle Zelle. Der Stapel wird in zwei parallel zu schaltenden Gruppen von Membrankammern zusammengefaßt, die von der Aufnehmer- bzw. der Abgeberlösung beschickt werden, wie in den Beispielen angegeben. Werden die Ströme im Kreis gefahren, so erfolgt eine fortlaufende Entsalzung. Sieht man aber ausreichende Membrankammern und Stapel (d. h. ausreichender Verfahrensweg) vor, so kann die Entsalzung auch in einem Durchlauf erfolgen.

Ein Gemisch aus Propargylalkohol, Butanol und Wasser gelangt als Strom 1 in den Elektrodialysestapel E und wird dort mit den Salzen Kupfer(I)chlorid und Ammoniumchlorid beladen. Der Strom entspricht somit dem Aufnehmerstrom. Der salzhaltige Strom 2 entspricht dem erforderlichen Reaktionsgemisch und wird in den Reaktor R geführt, wo die Umsetzung zu Hexadiin(2,4)-diol-1,6 stattfindet. Das Reaktionsgemisch wird als Strom 3 wieder in den Elektrodialysestapel geführt (Abgeberstrom), worin die Entsalzung stattfindet. Ströme 2 und 3 bilden einen Salzkreislauf.

Von der Elektrodialyse gelangt das Hexadiin(2,4)-diol-1,6 (Strom 4 in Abb. 1) zur Aufarbeitung bzw. Weiterverarbeitung, beispielsweise Hydrierung zu Hexandiol-1,6. In einer nachgeschalteten Kolonne kann man Hexandiol-1,6 und Butanol trennen. Das Butanol dient als Aufnehmerstrom für die Salze bei der Elektrodialyse und gelangt anschließend als Strom 1 über die Elektrodialyse zum Reaktor zur Umsetzung von Propargylalkohol. Die elektrodialytische Entsalzung kann man kontinuierlich, halbkontinuierlich oder diskontinuierlich durchführen, vorzugsweise arbeitet man kontinuierlich, in besonderen Fällen kann auch die diskontinuierliche Fahrweise von Vorteil sein.

Weitere Einzelheiten des erfindungsgemäßen Verfahrens erläutern die Beispiele.

Beispiel 1,1

In einem 2-1-Glasautoklaven, ausgerüstet mit Rührer, Thermometer und Druckhalteventil wird eine Lösung von 92,5 g Kupfer(I)chlorid und 300 g Ammoniumchlorid in 1000 g Wasser vorgelegt. Zu dieser Katalysatormischung gibt man eine Lösung von 140,3 g Propargylalkohol in 500 g Butanol. Die Reaktionsmischung wird unter Rühren (700 U/min) auf 30 °C aufgeheizt und durch Einleiten von Sauerstoff stickstofffrei geblasen. Dann wird ein Reaktionsdruck $p_e$ von 0,5 bar (Überdruck) eingestellt. Die Reaktionswärme wird durch Wasserkühlung abgeführt. Nach 100 min ist die Reaktion beendet (Propargylalkoholgehalt < 0,1). Der Reaktor wird entspannt, der Rührer abgestellt und die Butanolphase (700 g) abgesaugt. Die Umsetzung von Propargylalkohol zu Hexadiindiol ist nahezu quantitativ.
Zur Entsalzung gibt man die Butanolphase in eine Elektrodialyse.

Beispiel 1,2 (Abb. 1 bis 4, Tab. 1 bis 3)

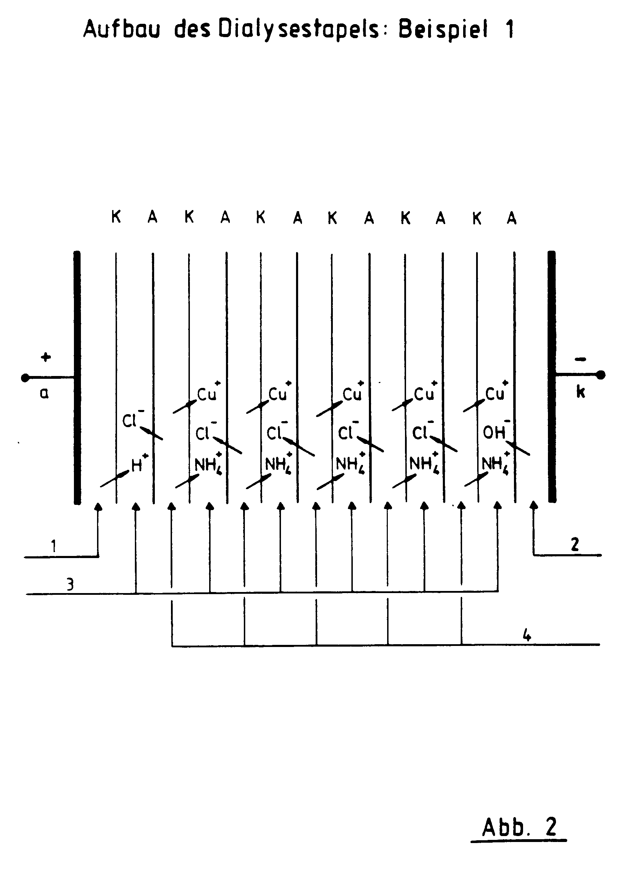

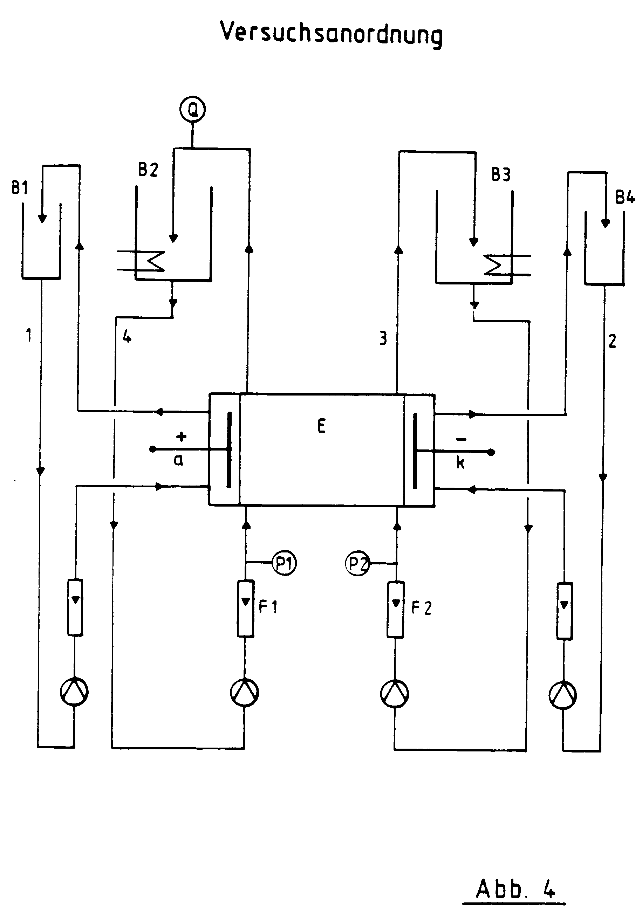

Der Elektrodialysestapel enthält alternierende Kationen- (K) und Anionenaustauschermembranen (A), mit einer Kationen- bzw. Anionenaustauschermembrane neben der Anode (a) bzw. Kathode (k). Die Elektroden werden mit 1 Gewichtsprozent H$_2$SO$_4$ (1) bzw. 0,5gewichtsprozentiger NaOH (2) umspült (s. Abb. 2). Der Stapel enthält 6 Membrankammern für die Aufnehmerlösung (3) sowie 5 Kammern für das Reaktionsgemisch (4). Die Leitfähigkeit des Reaktionsgemisches wird über die Dauer mit der Zelle Q verfolgt (s. Abb. 4). Die Zusammensetzungen der Lösungen sowie die technischen Daten sind in den Tabellen 1 und 3 zusammengestellt. Nach 5 Std. 45 Min. sind 93 % der Salze (auf Chlorbasis gerechnet) bei einer durchschnittlichen Stromausbeute von 80 % überführt worden. Nach weiteren 4 Std. 50 Min. entspricht die Entsalzung 97 % (Tab. 2).

Tabelle 1: Zur Versuchsanordnung

Membranstapel: Zellrahmen aus Hart-PVC bei Beispielen 1.2 und 2.2. Bei Beispiel 3 keine Rahmen.

Kationenaustauschermembran aus Styrol-Divinylbenzol mit angelagerten Sulfonat-Gruppen

Anionenaustauschermembran aus Styrol-Divinylbenzol mit angelagerten Aminogruppen

| | | |
|---|---|---|
| Kammerdicke | 3 mm | 0,4 mm |
| Membrandicke | 0,2 mm | 0,2 mm |
| effektive Fläche einer Membrane | 100 $cm^2$ | 220 $cm^2$ |
| | bei Beisp. 1.2 und 2.2 | bei Beisp. 3 |

| Beispiel | | 1,2 | 2,2 | 3 |
|---|---|---|---|---|
| Anzahl der Membrane | Kationenaustauscher | 5 | 7 | 25 |
| | Anionenaustaucher | 5 | 6 | 26 |
| gesamte wirksame Membranfläche ($m^2$) | | 0,10 | 0,12 | 0,55 |
| Anolyt | | 1 %ige $H_2SO_4$ | 1 %iges $NH_4Cl$ | 1 %iges $NH_4Cl$ |
| Katholyt | | 0,5 %ige NaOH | 1 %iges $NH_4Cl$ | 1 %iges $NH_4Cl$ |

Tabelle 2:    Zum Beispiel 1,2

Salzgehalt im Ausgangsgemisch              212,5 mval; Konzentration s. Tab. 3

Volumenstrom der Kreisläufe             230 1/h (= 5,6 cm/s)

Volumenstrom in den Elektrolyträumen     70 bzw. 115 1/h

Temperatur aller Kreisläufe             30 °C

Druck vor Membranstapel                1,3 bar

Spannung über gesamten Stapel u. Versuchsdauer   44,5 V

| Versuchs-dauer (min) | Spez. Leitfähig-keit σ vom Aus-gangsgemisch (μS/cm) | Strom I (mA) | Umsatz bis zum jeweiligen Zeitpunkt (mval) | (%) |
|---|---|---|---|---|
| 0 | 600 | - | | |
| 30 | 455 | 312 | | |
| 51 | 433 | 316 | | |
| 75 | 385 | 318 | | |
| 90 | 332 | 307 | | |
| 160 | 222 | 259 | | |
| 218 | 138 | 197 | | |
| 265 | 97 | 158 | | |
| 305 | 70,8 | 128 | | |
| 345 | 53,3 | 108,6 | 198,5 | 93,4 |
| 380 | 42,2 | 93,5 | | |
| 395 | 28,2 | 75,3 | | |
| 455 | 24,0 | 70,2 | | |
| 550 | 21,4 | 65,7 | | |
| 600 | 20,9 | 64,0 | | |
| 635 | 20,7 | 64,9 | 205,9 | 96,9 |

5

Tabelle 3: Anlysenergebnisse zum Beispiel 1,2

Reaktionsgemisch: 20 Gewichtsprozent Diindiol

70 Gewichtsprozent Butanol

10 Gewichtsprozent Wasser

Aufnehmerlösung: 70 Gewichtsprozent Butanol

20 Gewichtsprozent Propargylalkohol

10 Gewichtsprozent Wasser

| | Versuchs-dauer (min) | Konzentration (Gew.-ppm) | | |
| --- | --- | --- | --- | --- |
| | | $NH_4^+$ | Cu | Cl |
| Reaktionsgemisch | 0 | 1 300 | 400 | 2 600 |
| | 345 | 85 | 65 | 171 |
| | 635 | 60 | 40 | 82 |
| Aufnehmer-Kreislauf *) | 0 | 1 680 | 0 | 3 320 |
| | 345 | 1 700 | 110 | 4 600 |
| | 635 | 2 000 | 140 | 4 900 |

*) Um eine Leitfähigkeit im Aufnehmerkreislauf zu Versuchsbeginn zu gewährleisten, wurde hier eine 0,5 %ige $NH_4Cl$-Lösung vorgelegt.

Die $NH_4^+$-Werte sind sehr grob und lassen keine quantitative Aussage zu.

Beispiel 2,1

Durchführung wie Beispiel 1,1, jedoch wird anstelle von reinem Sauerstoff Luft als Sauerstofflieferant eingesetzt. Es wird deshalb ein kontinuierlicher Abgasstrom von 20 l/h gefahren. Die Reaktionszeit beträgt 685 min. Die Umsetzung verläuft nahezu quantitativ.

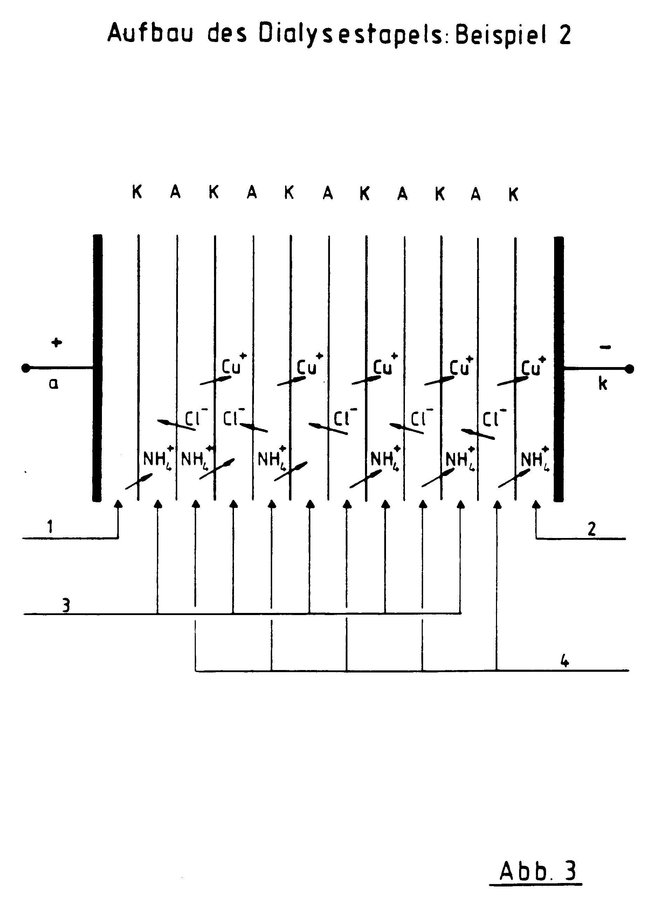

Beispiel 2,2 (Abb. 3 und 4, Tab. 1, 4 und 5)

In diesem Beispiel wird die Überführung von $H^+$- bzw. $OH^-$-Ionen aus den Ano- bzw. Katholytkammern nach Elektrolyse von Wasser vermieden. Es wird in beiden Elektrodenkreisläufen (1 und 2) eine 1gewichtsprozentige Ammoniumchloridlösung vorgelegt. Es findet nur eine Verschiebung der $NH_4^+$- $Cu^+$- und $Cl^-$-Ionen statt. (Der Nachteil, daß das $Cu^+$-Ion in das Katholyt verloren geht, kann bei einer großen Anzahl der Zellgruppen zwischen den Elektroden minimiert werden). Der Stapel besteht aus jeweils 5 Kammern für den Aufnehmer bzw. das Ausgangsgemisch. Nach 6 Std. 50 Min. sind 75 % der Salze (Chlorbasis) bei einer durchschnittlichen Ausbeute von 62 % überführt worden. Nach weiteren 15 Std. 46 Min. entspricht die Entsalzung 96 % (Tab. 4).

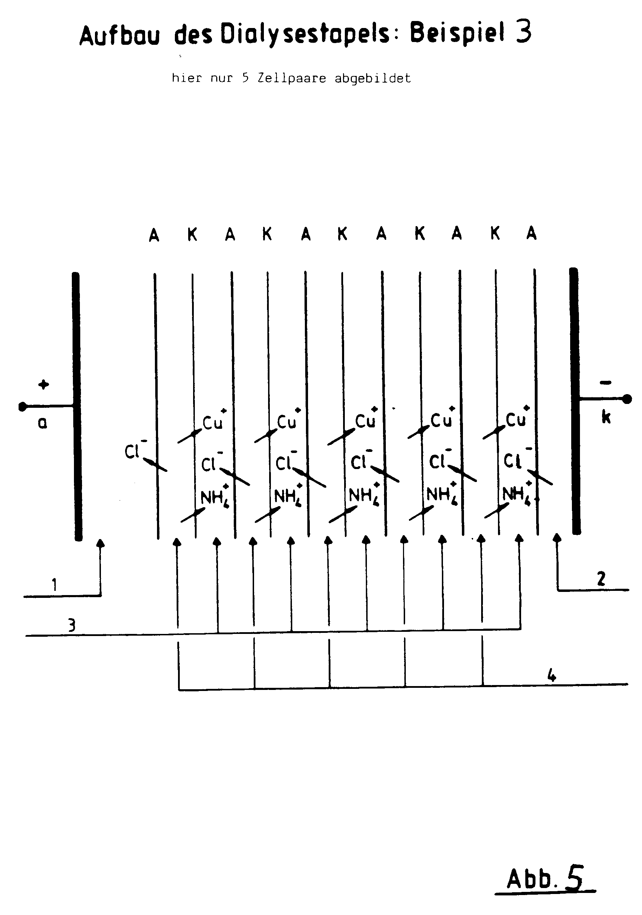

Beispiel 3 (Abb. 6 und 7, Tab. 1, 6 und 7)

Die Umsetzung verlief wie unter Beispiel 2,1, jedoch wurde ein anderer Elektrodialysestapel gewählt und zwar mit einer Zolldicke von 0,4 mm und einer Membranfläche von 220 cm$^2$. Auch die Membrananordnung wurde so gewählt, daß die Cu$^+$-Ionen in den beiden Kreisläufen erhalten werden (Abb. 4). Die Elektrodenspüllösungen kamen aus derselben Quelle, d. h. die beiden Kreisläufe hatten ein und denselben Vorratsbehälter. Zunächst wurde nur eine Hälfte der verfügbaren Aufnehmerlösung, nämlich 4 136 g, eingesetzt. Nach ca. 200 min wurde die Anlage gereinigt und das Diluat (8 403 g) weiter mit Hilfe der verbleibenden frischen Hälfte der Aufnehmerlösung entsalzt. In kürzerer Dauer konnte eine sehr niedrige Salzkonzentration im Diluat erzielt werden.

Tabelle 4:    Zum Beispiel 2,2

Salzgehalt im Ausgangsgemisch                         355 mval; Konzen-
                                                       tration s. Tab. 5

Volumenstrom der Kreisläufe                            230 1/h ($\hat{=}$ 5,6 cm/s)
Volumenstrom in den Elektrolyträumen                  80 bzw. 150 1/h
Temperatur aller Kreisläufe                            30 °C
Druck vor Membranstapel                               1,3 bar
Spannung über gesamten Stapel u. Versuchsdauer        45 V

| Versuchs-dauer (min) | Spez. Leitfähig-keit $\delta$ vom Aus-gangsgemisch ($\mu$S/cm) | Strom I (mA) | Umsatz bis zum jeweiligen Zeitpunkt (mval) | (%) |
|---|---|---|---|---|
| 0 | 473 | 277,5 | | |
| 6 | 466 | 287,6 | | |
| 36 | 447 | 294,5 | | |
| 90 | 417 | 292,5 | | |
| 120 | 388 | 294,0 | | |
| 150 | 355 | 288,6 | | |
| 230 | 292 | 283,6 | | |
| 280 | 243 | 264,2 | | |
| 340 | 199 | 237,8 | | |
| 400 | 165,1 | 210 | | |
| 410 | | | 266,6 | 75,1 |
| 442 | 137,7 | 191,3 | | |
| 540 | 95,1 | 144,6 | | |
| 750 | 44,7 | 85,5 | | |
| 990 | ↑ | 62,7 | | |
| 1 110 | defekt | 56,1 | | |
| 1 230 | ↓ | 55,4 | | |
| 1 356 | 25,0 | 52,5 | 341,4 | 96,2 |
| 1 410 | 26,6 | 53,5 | | |
| 1 470 | 26,9 | 54,1 | | |
| 1 530 | 27,2 | 54,1 | | |
| 1 590 | 27,2 | 53,6 | 343,7 | 96,8 |

Tabelle 5: Anlysenergebnisse zum Beispiel 2,2

Reaktionsgemisch und Aufnehmerlösung wie beim Beispiel 1,2

| | Versuchs-dauer (min) | Konzentration (Gew.-ppm) | | | |
|---|---|---|---|---|---|
| | | $NH_4^+$ | Cu (a) | (b) | Cl |
| Ausgangsgemisch | 0 | 1 180 | 280 | 340 | 2 350 |
| Beginn 5 337 g | 410 | 990 | 120 | 140 | 585 |
| Ende 4 895 g | | | | | |
| | 1 356 | 60 | 45 | 40 | 90 |
| | 1 590 | 20 | 30 | 40 | 75 |
| Aufnehmer-kreislauf | 0 | 1 180 | 5 | 6 | 2 870 |
| Beginn 4 996 g | 410 | 1 690 | 40 | 130 | 4 400 |
| Ende 5 394 g | 1 356 | 1 590 | 150 | 210 | 4 540 |
| | 1 590 | 1 580 | 160 | 200 | 4 430 |

(a) und (b): Analysen in zwei verschiedenen analytischen Labors

EP 0 297 239 B1

Tabelle 6:   Zum Beispiel 3

Salzgehalt im Ausgangsgemisch                          2 260 mval; Konzen-
                                                       tration s. Tab. 7

Volumenstrom der Kreisläufe                            280 l/h (= 13 cm/s)

Volumenstrom in den Elektrolyträumen                  530 l/h

Temperatur aller Kreisläufe                            25 °C

Druck vor Membranstapel                               1,25 bar

Spannung über gesamten Stapel u. Versuchsdauer        75 V

| Versuchs-dauer (min) | Spez. Leitfähig-keit $\delta$ vom Aus-gangsgemisch ($\mu$S/cm) | Strom I (mA) | Umsatz bis zum jeweiligen Zeitpunkt (mval) | (%) |
|---|---|---|---|---|
| 0 | 1 110 | - | | |
| 9 | 1 082 | 1 974 | | |
| 12 | 1 045 | 1 679 | | |
| 22 | 858 | 1 460 | | |
| 42 | 326 | 1 260 | | |
| 57 | 143 | 566 | | |
| 77 | 49 | 260 | | |
| 112 | 20,1 | 134 | | |
| 132 | 18,4 | 119 | | |
| 197 | 14,5 | 98 | 73 | 96,8 |
| 203 | 17,4 | 90 | | |
| 209 | 15,6 | 81 | | |
| 213 | 13,3 | 73 | | |
| 219 | 10,0 | 68 | | |
| 234 | 7,3 | 58 | | |
| 257 | 4,8 | 50 | | |
| 282 | 3,9 | 45 | | |
| 312 | 3,3 | 43 | | |
| 342 | 3,0 | 43 | 61 | 97,3 |

Tabelle 7:  Anlysenergebnisse zum Beispiel 3

Reaktionsgemisch:  1,4 Gewichtsprozent Propargylalkohol

9,2 Gewichtsprozent Diindiol

75,4 Gewichtsprozent Butanol

14  Gewichtsprozent Wasser

Aufnehmerlösung:  71  Gewichtsprozent Butanol

15  Gewichtsprozent Propargylalkohol

14  Gewichtsprozent Wasser

| | Versuchs-dauer (min) | Konzentration (Gew.-ppm) | | |
|---|---|---|---|---|
| | | $NH_4^+$ | Cu | Cl |
| Reaktionsgemisch | 0 | 1 900 | 2 600 | 5 500 |
| | 197 | 90 | 130 | 58 |
| | 342 | 90 | 100 | 24 |
| Aufnehmer-Kreislauf | 0 | 50 | 0 | 64 |
| | 197 | 2 900 | 3 200 | 9 400 |
| Einsatz nach 197 min | 342 | ≤50 | 60 | 405 |

Die $NH_4^+$-Werte sind sehr grob und lassen keine quantitative Aussage zu.

**Patentansprüche**

1. Verfahren zur Herstellung von Hexadiin(2,4)-diol-1,6 durch oxidative katalytische Kupplung von Propargylalkohol,
   dadurch gekennzeichnet,
   daß man der Reaktionsmischung aus Katalysator Propargylalkohol und Wasser Butanol zusetzt, die Reaktionsmischung auf 5 bis 80 °C bringt, unter intensivem Rühren Sauerstoff bei einem Überdruck $p_e$ auf 0,1 bis 5 bar aufdrückt, nach der Reaktion das zweiphasige Gemisch in eine butanolische Lösung von Hexadiin(2,4)-diol-1,6 und eine wäßrige Katalysatorlösung trennt und die butanolische Lösung als Abgeberstrom in die Elektrodialyse gibt, in die man als Aufnehmerstrom ein Gemisch aus Propargylalkohol, Butanol und Wasser gibt und aus der heraus man das entsalzte Hexadiin(2,4)-diol-1,6 als Produktstrom erhält sowie einen salzhaltigen Strom, der außer dem Katalysatorsalz Propargylalkohol, Wasser und Butanol enthält und damit dem Reaktionsgemisch entspricht, das man in den Reaktor zurückfährt.

2. Verfahren nach Anspruch 1,
   dadurch gekennzeichnet,
   daß man als Butanol n-, iso- oder tert.-Butanol einsetzt.

11

**3.** Verfahren nach Anspruch 1 und 2,
dadurch gekennzeichnet,
daß man ein Verhältnis von Katalysatorphase zu Butanolphase von 5 : 1 bis 1 : 5, vorzugsweise 3 : 1 bis 1 : 2 einstellt.

**4.** Verfahren nach einem der Ansprüche 1 bis 3,
dadurch gekennzeichnet,
daß man als Katalysator einen $Cu^I$-Cl/$NH_4$Cl-Komplex einsetzt.

**5.** Verfahren nach einem der Ansprüche 1 bis 4,
dadurch gekennzeichnet,
daß man die Elektrodialyse bei 10 bis 40 °C, vorzugsweise 20 bis 30°C durchführt.

**6.** Verfahren nach einem der Ansprüche 1 bis 5,
dadurch gekennzeichnet,
daß man das Verfahren kontinuierlich oder diskontinuierlich durchführt.

## Claims

**1.** A process for the preparation of hexa-2,4-diyne-1,6-diol by oxidative catalytic coupling of propargyl alcohol, characterised in that butanol is added to the reaction mixture of catalyst, propargyl alcohol and water, the reaction mixture is brought to 5 - 80°C, oxygen under a superatmospheric pressure $p_e$ of 0.1 - 5 bar is applied, with intensive stirring, after the reaction the two-phase mixture is separated into a solution of hexa-2,4-diyne-1,6-diol in butanol and an aqueous catalyst solution and the butanol solution is fed, as a donor stream, into an electrodialysis into which a mixture of propargyl alcohol, butanol and water is fed as the acceptor stream, and from which there is obtained the desalinated hexa-2,4-diyne-1,6-diol as the product stream, as well as a salt-containing stream which in addition to the catalyst salt contains propargyl alcohol, water and butanol and accordingly corresponds to the reaction mixture and which is recycled to the reactor.

**2.** A process according to claim 1, characterised in that n-, iso- or tert-butanol is employed as the butanol.

**3.** A process according to claim 1 and 2, characterised in that a ratio of catalyst phase to butanol phase of 5:1 to 1:5, preferably 3:1 to 1:2, is set up.

**4.** A process according to one of claims 1 to 3, characterised in that a $Cu^I$-Cl/$NH_4$Cl complex is employed as the catalyst.

**5.** A process according to one of claims 1 to 4, characterised in that the electrodialysis is carried out at 10 to 40°C, preferably 20 to 30°C.

**6.** A process according to one of claims 1 to 5, characterised in that the process is carried out continuously or batchwise.

## Revendications

**1.** Procédé de préparation d'hexa-di-yne-(2,4)-diol-1,6 par copulation catalytique oxydante d'alcool propargylique,
caractérisé par le fait que l'on ajoute du butanol au mélange réactionnel constitué par le catalyseur, l'alcool propargylique et l'eau, que l'on porte le mélange réactionnel à une température de 5 à 80°C, qu'en agitant de façon intensive, on envoie dessus de l'oxygène sous pression, à une surpression $p_e$ de 0,1 à 5 bars, qu'après la réaction on sépare le mélange à deux phases en une solution butanolique d'hexa-di-yne-(2,4)-diol-1,6 et en une solution aqueuse de catalyseur, puis que l'on ajoute la solution méthanolique, comme courant de donneur, dans l'électrodialyse, dans laquelle on ajoute, comme courant d'accepteur, un mélange d'alcool propargylique, de butanol et d'eau, et à partir de laquelle on obtient, comme produit dessalé, l'hexa-di-yne-(2,4)-diol-1,6 en tant que courant de produit, ainsi qu'un courant salin qui, outre le sel de catalyseur, renferme l'alcool propargylique, l'eau et le butanol et correspond par suite au mélange réactionnel que l'on renvoie dans le réacteur.

2. Procédé selon la revendication 1,
caractérisé par le fait que l'on utilise, comme butanol, le butanol normal, l'isobutanol ou le tertio-butanol.

3. Procédé selon les revendications 1 et 2,
caractérisé par le fait qu'entre la phase de catalyseur et la phase de butanol, on établit un rapport de 5 : 1 à 1 : 5, de préférence de 3 : 1 à 1 : 2.

4. Procédé selon l'une des revendications 1 à 3,
caractérisé par le fait que l'on utilise comme catalyseur un complexe de $CU^I$-Cl/$NH_4$Cl.

5. Procédé selon l'une des revendications 1 à 4,
caractérisé par le fait que l'on effectue l'électrodialyse à une température de 10 à 40°C, de préférence de 20 à 30°C.

6. Procédé selon l'une des revendications 1 à 5,
caractérisé par le fait que l'on met le procédé en oeuvre de façon continue ou discontinue.

Abb. 1

14

# Aufbau des Dialysestapels: Beispiel 1

**Abb. 2**

# Aufbau des Dialysestapels: Beispiel 2

## Abb. 3

# Versuchsanordnung

Abb. 4

# Aufbau des Dialysestapels: Beispiel 3

hier nur 5 Zellpaare abgebildet

A   K   A   K   A   K   A   K   A   K   A

+
a

Cu⁺   Cu⁺   Cu⁺   Cu⁺   Cu⁺

Cl⁻   Cl⁻   Cl⁻   Cl⁻   Cl⁻   Cl⁻

NH₄⁺   NH₄⁺   NH₄⁺   NH₄⁺   NH₄⁺

−
k

1

2

3

4

## Abb. 5